# EUROPÄISCHE PATENTSCHRIFT

(11) **EP 2 938 310 B1**
(45) Veröffentlichungstag und Bekanntmachung des Hinweises auf die Patenterteilung: **11.04.2018**
(21) Anmeldenummer: 13807980.1
(22) Anmeldetag: 13.12.2013
(51) Int. Cl.: A61F 13/537, A61F 13/15, D04B 21/10

(54) **FLÜSSIGKEITSVERTEILSCHICHT FÜR INKONTINENZPRODUKTE**
WICKING LAYER FOR INCONTINENCE PRODUCT
COUCHE À EFFET DE MÈCHE POUR PRODUIT D'INCONTINENCE

(30) Priorität: 28.12.2012 DE 102012113194
(43) Veröffentlichungstag der Anmeldung: 04.11.2015
(73) Patentinhaber: Müller Textil GmbH, 51674 Wiehl-Drabenderhöhe (DE)
(72) Erfinder: MÜLLER, Stefan, 51674 Wiehl (DE); MÜLLER, Frank, 51674 Wiehl (DE)
(74) Vertreter: Lorenz, Bernd Ingo Thaddeus
(86) Internationale Anmeldenummer: PCT/EP2013/076599
(87) Internationale Veröffentlichungsnummer: WO 2014/102072

(56) Entgegenhaltungen:
- EP-A2- 2 514 862
- WO-A1-2005/051656
- WO-A1-2012/153131
- DE-A1- 10 055 902
- DE-B3-102008 020 287
- DE-T2- 60 009 306
- US-A- 5 735 145

## Beschreibung

Die vorliegende Erfindung betrifft eine Flüssigkeitsverteilschicht für Inkontinenzprodukte gemäß dem Oberbegriff des Anspruches 1.

Flüssigkeitsaufnehmende Wegwerfprodukte werden in großen Mengen im Bereich der Körperhygiene eingesetzt. Neben Babywindeln und der Monatshygiene von Frauen stellt gerade der Bereich von Inkontinenzprodukten für Erwachsene einen wesentlichen Markt dar, wobei in den verschiedenen Bereichen stets unterschiedliche spezifische Anforderungen zu berücksichtigen sind. Die Inkontinenz von Erwachsenen kann auf unterschiedlichen Ursachen beruhen. Neben einem altersbedingten Kontrollverlust kann eine Inkontinenz auch als Begleiterscheinung anderer Krankheiten oder auch bei Frauen nach einer Schwangerschaft auftreten.

Gerade im Bereich der Inkontinenzprodukte für Erwachsene ergeben sich spezielle, hohe Anforderungen. Anders als bei Baby-Windeln sollen die als saugende Pats oder vorzugsweise in Form von Höschenwindeln vorgesehenen Produkte möglichst unsichtbar sein, damit die Beeinträchtigungen des Benutzers von anderen nicht erkannt werden können. Gerade wenn die Kontrolle über die Blasenfunktion überhaupt nicht mehr vorhanden ist, ist auch ein sehr großes Flüssigkeitsaufnahmevermögen notwendig, um bei der Benutzung die notwendige Sicherheit zu gewährleisten. Schließlich soll das beschriebene Hygieneprodukt auch einen guten Tragekomfort aufweisen und eine Beeinträchtigung der Haut des Benutzers verhindern.

Um den speziellen Anforderungen gerecht zu werden, wird in der Regel ein mehrschichtiger Aufbau eines Hygieneproduktes vorgesehen, wobei zusätzlich zu einer wasserdichten Außenschicht und zumindest einer flüssigkeitsaufnehmenden Schicht auch eine Flüssigkeitsverteilschicht vorhanden ist. Die Flüssigkeitsverteilschicht ist insbesondere dazu notwendig, um bei einer möglichst dünnen, unsichtbaren Form des Hygieneartikels die zum Teil großen auftretenden Flüssigkeitsmengen über eine größere Fläche zu verteilen. Bei einer dünnen flüssigkeitsaufnehmenden Schicht kann nämlich die von einem Benutzer abgegebene Flüssigkeiten nicht vollständig an der entsprechenden Stelle aufgenommen werden und muss innerhalb des Hygieneartikels verteilt werden.

Selbstverständlich soll trotz dieser verteilten Flüssigkeitsaufnahme keinerlei Flüssigkeit an die Umgebung abgegeben werden, wobei auch die Haut des Benutzers möglichst trocken gehalten werden soll. Gerade bei dem bevorzugten Einsatz von superabsorbierenden Polymeren in der Saugschicht ist eine Flüssigkeitsverteilung über eine größere Fläche notwendig, weil ansonsten lokale Aufquellungen auftreten können.

Aus der EP 1 330 225 B1 ist ein absorbierender Einweg-Artikel mit einer Flüssigkeitsverteilschicht bekannt, wobei diese Verteilschicht eine gewisse Beweglichkeit und Elastizität aufweist. Durch diese Beweglichkeit und Elastizität kann eine die Flüssigkeitsverteilung befördernde Pumpwirkung erreicht werden. Als Fluidverteilschicht ist ein Vliesmaterial vorgesehen. Ein Hygieneartikel mit einer Flüssigkeitsverteilschicht ist aus der DE 692 22 374 T2 bekannt.

Hygieneartikel wie Inkontinenzprodukte werden in der Praxis zum allergrößten Teil als Einweg-Wegwerfartikel produziert und eingesetzt. Bei Mehrweg-Artikeln ergibt sich das Problem einer aufwendigen Reinigung, wobei gerade die unterschiedlichen Schichten schwierig zu reinigen sind. Dies gilt insbesondere auch für die Saugeinlage, die bei der Benutzung große Mengen an Flüssigkeit aufnehmen und festhalten soll. Bei einer Reinigung muss aber auch die Sauglage von Verunreinigungen befreit und getrocknet werden, was genau gegenläufig zu den Eigenschaften bei der Benutzung ist.

Aus der WO 2005/051656 A1 ist ein Inkontinenzprodukt für eine mehrfache Benutzung beschrieben, welches eine Flüssigkeitsverteilschicht mit den eingangs beschriebenen Merkmalen aufweist. Als Flüssigkeitsverteilschicht ist ein Abstandsgewirke vorgesehen, welches eine erste flächige Gewirkelage, eine zweite flächige, ein Muster von Öffnungen aufweisende Gewirkelage und Abstandsfäden aufweist. Aus der WO 2005/051656 A1 ist damit ein hochwertiges, widerstandsfähiges Hygieneprodukt bekannt, welches mehrfach benutzt und gereinigt werden kann. Bei dem Abstandsgewirke handelt es sich um ein vergleichsweise aufwendiges, teures Material, welches insbesondere aus Kostengründen nicht für Einweg-Hygieneartikel eingesetzt wird. Die erste Gewirkelage und die zweite Gewirkelage sind aus unterschiedlichen Fäden gebildet, um einerseits hydrophobe und andererseits hydrophile Eigenschaften erreichen zu können. Darüber hinaus sollen auch Kapillarwirkungen genutzt werden, wozu enge Zwischenräume des Materials notwendig sind und das Material, das heißt die Fäden entsprechend mit einer ausreichenden Dichte angeordnet werden müssen. Die hohen Herstellungskosten für ein entsprechend speziell angepasstes Material sind bei einem Wegwerfartikel nicht akzeptabel.

Aus der DE 600 34 681 T2 ist ein Abstandstextil für eine Matratzenauflage bekannt, welches sich durch eine Dochtwirkung auszeichnet. Die mechanischen Eigenschaften des Abstandstextils sind im Hinblick auf den Einsatz als Matratzenauflage und die dabei zu erwartenden Belastungen abgestimmt.

Vor diesem Hintergrund liegt der vorliegenden Erfindung die Aufgabe zugrunde, eine Flüssigkeitsverteilschicht für Inkontinenzprodukte anzugeben, die sich durch hervorragende Funktionseigenschaften auszeichnet und dennoch kostengünstig produziert werden kann, und insbesondere auch für Einweg-Inkontinenzartikel wirtschaftlich einzusetzen ist.

Gegenstand der Erfindung und Lösung der Aufgabe ist eine Flüssigkeitsverteilschicht für Inkontinenzprodukte gemäß Patentanspruch 1. Erfindungsgemäß wird eine Flüssigkeitsverteilschicht mit einer ersten flächigen Gewirkelage, mit einer zweiten flächigen, ein Muster von Öffnungen aufweisenden Gewirkelage und mit Abstandsfäden, welche die erste Gewirkelage und die zweite Gewirkelage miteinander verbinden, vorgegeben, wobei die Gesamtdicke mehr als 3 mm und weniger als 6 mm beträgt, wobei die Öffnungen in einem Rautenmuster angeordnet sind, wobei das Flächengewicht weniger als 300 g/m² beträgt und wobei die Abstandsfäden eine Feinheit zwischen 80 und 130 dtex aufweisen und in einer Dichte zwischen 65 und 110 pro Quadratzentimeter (/cm2) angeordnet sind. Die Dicke bzw. Gesamtdicke der Flüssigkeitsverteilschicht wird insbesondere nach der Norm EN ISO 5084 vorzugsweise bei einer Beaufschlagung mit einer Vorkraft von 1 Kilopascal (kPa) gemessen. Zweckmäßigerweise beträgt die Gesamtdicke 4,0 mm bis 4,5 mm und gemäß einer bevorzugten Ausführungsform 4,3 mm bzw. ungefähr 4,3 mm.

Erfindungsgemäß wird damit eine Flüssigkeitsverteilschicht angegeben, die trotz eines geringen Flächengewichtes und durch die beschriebene Auswahl und Anordnung der Abstandsfäden bei einer Gesamtdicke zwischen 3 und 6 mm überraschenderweise noch eine ausreichende Stabilität und Fähigkeit zur Verteilung von Flüssigkeit aufweist, um als Flüssigkeitsverteilschicht in einem Inkontinenzprodukt eingesetzt zu werden. Erfindungsgemäß wird damit eine konkrete, enge Kombination aufeinander abgestimmter Parameter angegeben, welche im besonderen Maße auf den Einsatz als Flüssigkeitsverteilschicht in einem Inkontinenzprodukt abgestimmt sind. Im Vordergrund steht dabei, dass auch unter Belastungen die Flüssigkeit in der Ebene des Abstandstextils über einen großen Bereich verteilt werden kann.

Es liegt im Rahmen der Erfindung, dass das Flächengewicht weniger als 260 g/m² beträgt. Das Flächengewicht kann beispielsweise 150g/m² bis 300 g/m², insbesondere zwischen 150 g/m² und 250 g/m², vorzugsweise zwischen 200 g/m² und 230 g/m² liegen.

Um die Herstellungskosten gering zu halten, wird vorzugsweise auf weitere Funktionalitäten wie eine unterschiedliche Affinität der Schichten zu Flüssigkeit insbesondere Wasser, durch eine hydrophobe bzw. hydrophile Ausgestaltung sowie auf eine den Flüssigkeitstransport unterstützende Kapillarwirkung verzichtet. Im Vordergrund steht im Rahmen der Erfindung die Eigenschaft, bei den typischen Belastungen in einem Inkontinenzprodukt den Abstand zwischen den beiden Gewirkelagen aufrechtzuerhalten, um die Verteilung über eine möglichst große Fläche zu ermöglichen. Eine saugende Wirkung der Flüssigkeitsverteilschicht ist in der Regel nicht vorgesehen und auch nicht gewünscht. Neben einem erhöhten Aufwand der mit der Erzeugung saugende Eigenschaften verbunden ist, soll die Flüssigkeitsverteilschicht insgesamt auch nur eine geringe Affinität zu wässrigen Flüssigkeiten aufweisen, weil die Aufnahme der Flüssigkeit bei der beschriebenen, bevorzugten Anwendung durch eine separate Saugschicht bereitgestellt wird.

Insbesondere können die erste und die zweite Gewirkelage sowie die Abstandsfäden aus dem gleichen oder zumindest einem ähnlichen polymeren Material bestehen. Besonders bevorzugt sind die erste und die zweite Gewirkelage sowie die Abstandsfäden aus Polyesterfäden gebildet, die hinsichtlich der jeweiligen Anforderungen optimiert sind.

Erfindungsgemäß weisen die Abstandsfäden eine Feinheit zwischen 80 und 130 dtex, vorzugsweise zwischen 100 und 120 dtex auf. Um bei einem minimierten Materialeinsatz ausreichende Staucheigenschaften der Flüssigkeitsverteilschicht zu ermöglichen, wird für die Abstandsfäden zweckmäßigerweise ein glattes Monofilamentgarn eingesetzt. Im Vergleich zu dem Stand der Technik gemäß der WO 2005/051656 A1 wird dabei die Funktion der Fluidverteilschicht auf das Bereitstellen einer elastischen Zwischenschicht beschränkt, wobei diese nicht weiter mit der Flüssigkeit in Wechselwirkung tritt. Durch den Einsatz von Multifilamentgarnen für die Abstandsfäden könnte zwar eine gewisse Kapillarwirkung erzeugt werden, jedoch würde der Einsatz eines solchen Materials dann entweder zu einer Reduzierung der Stauchhärte oder bei dem Einsatz entsprechend dickerer Fäden zu einem höheren Flächengewicht führen. Darüber hinaus wird mit Monofilamentgarn eine bessere Flüssigkeitsverteilung erreicht.

Im Gegensatz zu dem Stand der Technik erfolgt bei dem Einsatz an einem Inkontinenzprodukt eine noch klarer Aufteilung zwischen Flüssigkeitsverteilschicht und einer in der Regel vorgesehenen Saugschicht. Dadurch ergibt sich auch der Vorteil, dass bei der Benutzung in einem Inkontinenzprodukt keine Wesentlichen Mengen an Urin oder einer anderen Flüssigkeit in der Flüssigkeitsverteilschicht zurückbleiben.

Die in der zweiten Gewirkelage vorgesehenen Öffnungen ermöglichen einen schnellen Eintritt von Flüssigkeit in die Fluidverteilschicht. Gleichzeitig müssen zwischen den Öffnungen noch Stege der zweiten Gewirkelage verbleiben, in denen die Fäden der zweiten Gewirkelage verlaufen und in denen die Abstandsfäden eingebunden sind. Schließlich muss die zweite Gewirkelage auch insgesamt noch eine ausreichende Festigkeit aufweisen, wobei auch keine mikroskopische Strukturierung auftreten soll, die von einem Benutzer als störend empfunden werden kann.

Vor diesem Hintergrund ist gemäß einer bevorzugten Ausgestaltung vorgesehen, dass die Dichte der in der zweiten Gewirkelage vorgesehenen Öffnungen zwischen 3 und 6,5 besonders bevorzugt zwischen 4 und 5,5 pro Quadratzentimeter (/cm²) beträgt. Die Öffnungen können insbesondere eine Fläche zwischen 4,5 und 11,25 mm² aufweisen. Vorzugsweise liegt eine elliptische Form vor, wobei die lange Achse eine Länge zwischen 3,0 und 4,5 mm und die kurze Achse eine Länge zwischen 1,5 und 2,5 mm aufweisen kann.

Die erste Gewirkelage soll dazu dienen, die Flüssigkeit über einen größeren Bereich zu verteilen. Große Öffnungen könnten einen zu schnellen, lokalen Abfluss der Flüssigkeit bewirken, weshalb die erste Gewirkelage zweckmäßigerweise eine gleichmäßige Maschenstruktur in einer Produktionsrichtung und in einer Querrichtung aufweist. Gemäß einer bevorzugten Ausgestaltung der Erfindung ist vorgesehen, dass die erste Gewirkelage in der Produktionsrichtung zwischen 8 und 12 Maschenreihen pro Zentimeter (/cm) sowie in einer Querrichtung zwischen 7 und 10 Maschenstäbchen pro Zentimeter (/cm) aufweist.

Wie bereits eingangs erörtert, muss die Fluidverteilschicht ausreichende Rückstellkräfte haben, um bei der Belastung durch einen Benutzer nicht sofort zusammengedrückt werden bzw. um sich nach dem Wegfall einer Druckkraft wieder vollständig auszurichten. Andererseits darf die Fluidverteilschicht auch nicht zu hart sein, weil diese ansonsten von einem Benutzer als störend empfunden wird. Die erfindungsgemäße Flüssigkeitsverteilschicht mit den flächigen Gewirkelagen und den Abstandsfäden dazwischen ist vor diesem Hintergrund besonders gut geeignet, weil das Material ein relativ weiches Ansetzverhalten, das heißt eine gute Nachgiebigkeit zu Beginn der Komprimierung, und einen großen elastischen Bereich aufweist.

Wie zuvor bereits beschrieben, zeichnet sich die Flüssigkeitsverteilschicht durch eine leichte, offene Struktur aus. Dies führt auch dazu, dass die Flüssigkeitsverteilschicht ausgehend von einem unbelasteten Zustand in ihrer Dicke um üblicherweise mehr als 50 %, vorzugsweise mehr als 60 % komprimiert werden kann. Nach Wegfall der Druckkraft entspannt sich die Flüssigkeitsverteilschicht reversibel und gelangt so in ihre Ausgangslage.

Zur Charakterisierung der Eigenschaften der Flüssigkeitsverteilschicht kann eine Bestimmung der Druckspannungs-Verformungseigenschaften gemäß DIN EN ISO 3386-1:2010-09 erfolgen. Obwohl diese Norm die Bestimmung der Druckspannungs-Verformungseigenschaften von weichelastischen Schaumstoffen betrifft, kann auch eine Anwendung auf Abstandsgewirke erfolgen. Um für den Einsatz in Inkontinenzprodukten besonders vorteilhafte Eigenschaften aufzuweisen, beträgt bei der erfindungsgemäßen Flüssigkeitsverteilschicht der Druckspannungswert bei einer Verformung von 40 % CV₄₀ zwischen 4 und 8, vorzugsweise zwischen 5 und 6,5 Kilopascal (kPa). Des Weiteren kann vorgesehen sein, dass ausgehend von einer Verformung von 20 % bis zu einer Verformung von 50 % die Zunahme der Druckspannung zwischen 1,5 und 6, vorzugsweise zwischen 2 und 3,5 Kilopascal (kPa) liegt. Entsprechend wird ein großer Verformungsbereich mit gleichmäßigen elastischen Eigenschaften bereitgestellt.

Des Weiteren ist zu berücksichtigen, dass die Flüssigkeitsverteilschicht auch im komprimierten Zustand lagerfähig sein muss, wobei diese ihre Funktionalität nicht verlieren darf. So ist es üblich, dass Inkontinenzprodukte aus Platzgründen im komprimieren Zustand gelagert werden. Um die Lagerfähigkeit zu prüfen, wird ein Abschnitt der Flüssigkeitsverteilschicht mit einer Größe 10 x 10 cm mit einem Gewicht von 6 kg belastet und im komprimierten Zustand über eine Dauer von vier Wochen bei einer Temperatur von 40 °C gelagert. Nach Wegfall der Druckspannung dehnt sich die Flüssigkeitsverteilschicht wieder aus. Gemäß einer bevorzugten Ausgestaltung der Erfindung beträgt die Dicke nach sieben Tagen Verweilzeit mindestens 80% der Ausgangsdicke vor der Lagerung, wobei in der Praxis aufgrund der Struktur der Flüssigkeitsverteilschicht die Rückstellung bereits unmittelbar nach dem Wegfall der Spannung nahezu vollständig erfolgt.

Die erfindungsgemäße Flüssigkeitsverteilschicht zeichnet sich wie zuvor beschrieben durch eine gute, gleichmäßige Stauchhärte bei einem geringen Flächengewicht aus. Ein charakteristischer Parameter für das Material ist deshalb auch das Verhältnis der Dicke zu dem Flächengewicht des Materials unter einer Druckbelastung. Das genannte Verhältnis wird entsprechend in der Einheit m/(g/m²) (Dicke pro Flächengewicht) angegeben. Erfindungsgemäß ist das angegebene Verhältnis im Vergleich zu anderen Flüssigkeitsverteilschichten groß und ändert sich auch nur wenig in Abhängigkeit der Druckbelastung.

Um das druckabhängige Verhältnis von Dicke zu Flächengewicht zu bestimmen, wurde ein entsprechendes Testverfahren entwickelt. Gemäß dem Testverfahren wird ein Muster mit einer Kantenlänge von 50 mm bereitgestellt und mittig mit einem zylindrischen Stempel belastet, der einen Durchmesser von 10 mm aufweist. Der Stempel wird dann mehrfach in das Material hineingedrückt, wobei sowohl der Weg als auch die dazu notwendige Kraft aufgenommen werden. Ausgehend von dem zylindrischen Durchmesser des Stempels von 10 mm und der jeweils gemessenen Kraft kann der Druck (Kraft pro Fläche) in Pascal (Pa) bzw. Kilopascal (kPa) bestimmt werden. Die Vorschubgeschwindigkeit für den Test beträgt 5 mm pro Minute. Gemäß dem so durchgeführten Test beträgt das Verhältnis der Dicke zu dem Flächengewicht bei einem Druck von 5 Kilopascal zwischen 1,5 x 10⁻⁵ und 2,5 x 10⁻⁵ m/(g/m²). Vorzugsweise wird auch noch bei einem Druck von 10 Kilopascal (kPa) ein Verhältnis innerhalb des angegebenen Bereiches erreicht.

Erfindungsgemäß sind die Öffnungen in der zweiten Gewirkelage rautenförmig angeordnet. Damit ist gemeint, dass die in Produktionsrichtung aufeinanderfolgenden Reihen von Öffnungen in Querrichtung versetzt, insbesondere genau auf Lücke versetzt sind, so dass sich eine Rautenform ergibt. Die genaue Form der Raute hängt dabei von der Geometrie der Öffnungen ab.

Die Erfindung betrifft auch die Verwendung der Flüssigkeitsverteilschicht in einem Einweg-Inkontinenzprodukt, welches üblicherweise ein superabsorbierendes Polymer enthält. Bei dem Einsatz eines superabsorbierenden Polymers ist eine mehrfache Verwendung ausgeschlossen, weil das superabsorbierende Polymer die Flüssigkeit unter Aufquellen bindet, und somit nicht gewaschen werden kann.

Die flächigen Gewirkelagen werden vorzugsweise von einem texturierten Filament gebildet, wobei die Feinheit des Fadens für die erste Gewirkelage vorzugsweise zwischen 90 und 130 dtex und für den Faden der zweiten Gewirkelage vorzugsweise zwischen 60 und 100 dtex beträgt.

Die erfindungsgemäße Flüssigkeitsverteilschicht mit den flächigen Gewirkelagen und den Abstandsfäden dazwischen wird üblicherweise in einem Wirkprozess als breite Endlosbahn produziert, wobei dann einzelne Abschnitte zur Herstellung von Einweg-Inkontinenzprodukten abgeschnitten werden. Insbesondere kann die breite Materialbahn zunächst auch in Streifen geschnitten werden, bevor dann einzelne Abschnitte von den Streifen abgetrennt werden. Um in einem geeigneten Herstellungsverfahren die Flüssigkeitsverteilschicht mit anderen Materialien zu verbinden und zu kombinieren, wird die Materialbahn bzw. werden einzelne Streifen der Materialbahn üblicherweise auf- und abgewickelt sowie als Endlosmaterial unter Zug transportiert. Vor diesem Hintergrund ist für die erfindungsgemäße Flüssigkeitsverteilschicht auch eine gute Zugfestigkeit, das heißt eine geringe Dehnung zweckmäßig, wobei ein Auf- und Abwickeln sowie ein Transport üblicherweise entlang der Produktionsrichtung des textilen Materials erfolgen.

Die Zugeigenschaften von textilen Flächengebilden können gemäß der Norm EN ISO 13934-1:1999 bestimmt werden. Vorzugsweise beträgt die nach dieser Norm bestimmte Dehnung bei einer Zugkraft von 25 N weniger als 8 %, bevorzugt weniger als 6 %. Die Dehnung kann beispielsweise zwischen 2 und 7 %, beispielsweise bei etwa 3 % liegen.

Bei einer Zugkraft von 50 N beträgt die in Produktionsrichtung bestimmte Dehnung vorzugsweise weniger als 10 %. Die Dehnung kann beispielsweise zwischen 3 und 10 %, beispielsweise bei etwa 5 % liegen.

Bei einer Zugkraft von 100 N beträgt die Dehnung vorzugsweise weniger als 15 %. Die Dehnung kann beispielsweise zwischen 6 % und 14,5%, beispielsweise bei etwa 8,5 % liegen.

Die Erfindung wird im Folgenden anhand einer lediglich ein Ausführungsbeispiel darstellenden Zeichnung erläutert.

Es zeigen:
- **Fig. 1**: eine erfindungsgemäße Flüssigkeitsverteilschicht in schematischer Darstellung;
- **Fig. 2**: ein Spannungs-Verformungs-Diagramm für eine erfindungsgemäße Fluidverteilschicht.

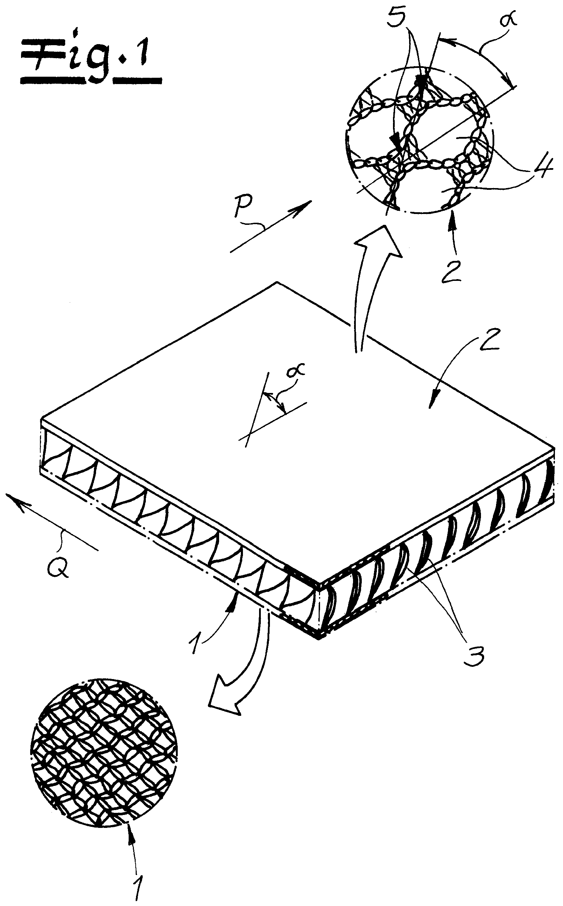

Die Fig. 1 zeigt eine Flüssigkeitsverteilschicht für Inkontinenzprodukte mit einer ersten flächigen Gewirkelage 1 und mit einer zweiten flächigen Gewirkelage 2, die durch Abstandsfäden 3 miteinander verbunden sind.

Die Abstandsfäden 3 sind aus einem Monofilamentgarn gebildet und in einer Produktionsrichtung P bogenförmig gekrümmt, wobei durch diese Ausgestaltung der Abstandsfäden 3 die beiden Gewirkelage 1, 2 elastisch federnd auf Abstand gehalten werden. Im unbelasteten Zustand beträgt die Dicke der Flüssigkeitsverteilschicht zwischen 3 mm und 6 mm.

Der Fig. 1 ist zu entnehmen, dass die zweite Gewirkelage 2 Öffnungen 4 aufweist, während die erste Gewirkelage 1 eine gleichmäßige Maschenstruktur mit vorzugsweise zwischen 8 und 12 Maschenreihen pro Zentimeter (/cm) in Produktionsrichtung P sowie zwischen 7 und 10 Maschenstäbchen pro Zentimeter (/cm) in Querrichtung Q aufweist. Grundsätzlich sind in der ersten Gewirkelage und der zweiten Gewirkelage bedingt durch den Wirkprozess die gleiche Anzahl an Maschenreihen vorgesehen. Da die zweite Gewirkelage mit Öffnungen versehen ist, sind die gebildeten Maschen nicht gleichmäßig homogen angeordnet sondern um die Öffnungen herum konzentriert.

In jede Masche der ersten Gewirkelage 1 ist sowohl in Querrichtung Q als auch in Produktionsrichtung P ein Abstandsfaden 3 eingewirkt, so dass die Abstandsfäden 3 vorzugsweise in einer Dichte zwischen 65 und 110 pro Quadratzentimeter (/cm²) angeordnet sind. Die Fäden für die erste Gewirkelage 1, die zweite Gewirkelage 2 und die Abstandsfäden 3 sind so ausgewählt, dass bei der angegebenen Gesamtdicke das Flächengewicht weniger als 260 g/m² beträgt, so dass ein leichtes aber dennoch ausreichend stauchfestes Material vorliegt, welches als Flüssigkeitsverteilschicht in einem Inkontinenzprodukt eingesetzt werden kann. Das Inkontinenzprodukt kann sowohl als Einlage als auch nach Art einer Höschenwindel ausgestaltet sein.

Die Öffnungen 4 sind in einer Rautenform angeordnet, wobei in Produktionsrichtung P aufeinanderfolgende Reihen von Öffnungen 4 zueinander genau auf Lücke angeordnet sind. Die Öffnungen 4 weisen in etwa eine elliptische, in Produktionsrichtung P erstreckte Form auf, so dass sich schräg zu der Produktionsrichtung verlaufende Stege 5 ergeben, die gegenüber der Produktionsrichtung P einen Winkel α von weniger als 45° aufweisen. Die Stege 5 können gegenüber der Produktionsrichtung P insbesondere einen Winkel α zwischen 20° und 40°, insbesondere zwischen 25° und 35°, beispielsweise etwa 30° einschließen. Die beschriebene Form ergibt sich aus einer Bereitstellung einer möglichst offenen Struktur, die aber auch eine große Stauchhärte aufweisen sollen.

Während die Abstandsfäden 3 in der ersten Gewirkelage 1 im Wesentlichen gleichmäßig eingebunden sind, sind diese an der zweiten Gewirkelage entlang der Stege 5 konzentriert. Dies hat zur Folge, dass sich zwischen den Stegen 5 schräg verlaufende Kanäle ergeben, an denen die Dichte der Abstandsfäden 3 reduziert ist. Durch die beschriebenen Kanäle kann ein verbesserter Transport von Flüssigkeit entlang der schrägen Kanäle erreicht werden.

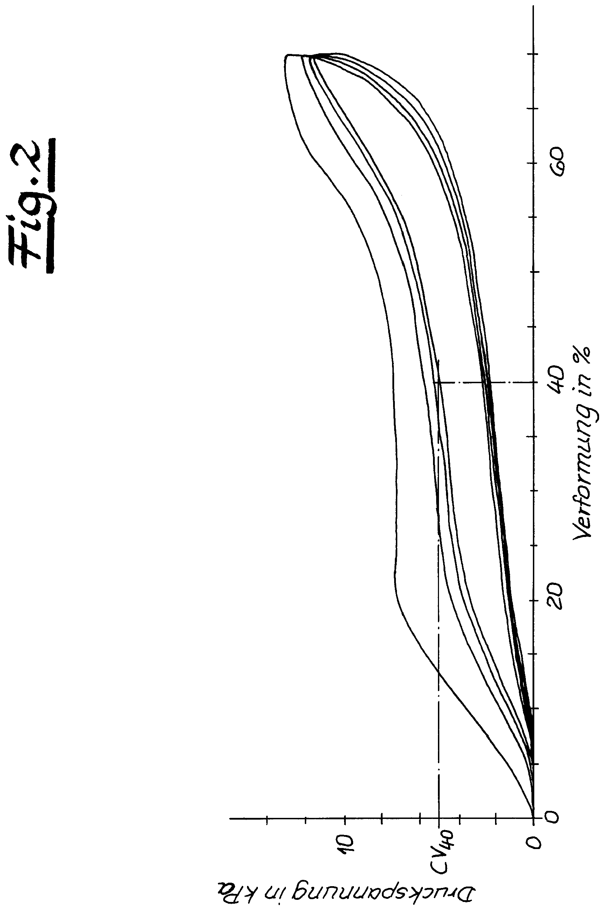

Die Fig. 2 zeigt ein Spannungs-Dehnungs-Diagramm, anhand welchem der Druckspannungswert CV₄₀ bei einer Verformung von 40 % gemäß DIN EN ISO 3386-1:2010-09 bestimmt wird. Die Messung erfolgte an einer Flüssigkeitsverteilschicht mit einem Flächengewicht von 206 g/m² und einer Dicke von 4,31 mm, wobei zu einer Erhöhung der Genauigkeit und einer Überprüfung der Gleichmäßigkeit des Materials Messungen an den Rändern sowie in der Mitte eines als Flüssigkeitsverteilschicht vorgesehenen Abstandsgewirkes vorgenommen wurden.

In der Fig. 2 ist zu erkennen, dass ausgehend von einem unbelasteten Material bei einer ersten Komprimierung erhöhte Druckspannungen bezogen auf die Verformung beobachtet werden. Diese erste Komprimierung stellt eine Art Aktivierung des Materials dar, wobei das Material bei dieser ersten Komprimierung eine erhöhte Steifigkeit aufweist. Ein solches Verhalten wird auch bei anderen elastischen Materialien, beispielsweise elastischen Folien, beobachten.

Nach einer erstmaligen Komprimierung ist die Druckspannungs-Verformungs-Charakteristik weitgehend reproduzierbar, wobei sich eine typische Hystereseform ergibt. Es ist zu erkennen, dass bis zu einer Verformung von etwa 20 % bei einer Komprimierung ein erster in etwa linearer Bereich vorhanden ist. Entsprechend kann die Flüssigkeitsverteilschicht auch mit einer geringeren Druckspannung zumindest etwas eingerückt werden. Bei einer weiteren Zunahme der Verformung ist die Steigung, das heißt der Anstieg der Druckspannung bis zu einer Verformung von etwa 50 % geringer. Schließlich folgt noch ein weiterer Bereich, in dem die Steigung bis zu der in den Untersuchungen maximalen Verformung von 70 % wieder erhöht ist. Aus dem Kurvenverlauf ergibt sich, dass das Material um zumindest 70 % komprimierbar ist, weil dort noch kein steiler, abrupter Anstieg der Druckspannung beobachtet wird, der Eintritt, wenn die beiden Gewirkelagen aufeinander gedrückt sind.

Der Druckspannungswert CV₄₀ bei einer Verformung von 40 % wird gemäß der DIN EN ISO 3386-1:201009 bei der Komprimierung in einem vierten Komprimierungszyklus bestimmt und beträgt in dem Ausführungsbeispiel etwa 5,55 Kilopascal (kPa).

## Patentansprüche

1. Flüssigkeitsverteilschicht für Inkontinenzprodukte mit einer ersten flächigen Gewirkelage (1), mit einer zweiten flächigen, ein Muster von Öffnungen (4) aufweisenden Gewirkelage (2) und mit Abstandsfäden (3), welche die erste Gewirklage (1) und die zweite Gewirklage (2) miteinander verbinden, wobei die Gesamtdicke mehr als 3 mm und weniger als 6 mm beträgt und wobei die Öffnungen (4) in einem Rautenmuster angeordnet sind, **dadurch gekennzeichnet, dass** das Flächengewicht weniger als 300 g/m² beträgt, wobei die Abstandsfäden (3) eine Feinheit zwischen 80 und 130 dtex aufweisen und in einer Dichte zwischen 65 und 110 pro Quadratzentimeter (/cm²) angeordnet sind.

2. Flüssigkeitsverteilschicht nach Anspruch 1, **dadurch gekennzeichnet, dass** die Dichte der in der zweiten Gewirkelage vorgesehenen Öffnungen zwischen 3 und 6,5 pro Quadratzentimeter (/cm²) beträgt.

3. Flüssigkeitsverteilschicht nach Anspruch 1 oder 2, **dadurch gekennzeichnet, dass** die erste und die zweite Gewirklage (1, 2) sowie die Abstandsfäden (3) aus Polyesterfäden gebildet sind.

4. Flüssigkeitsverteilschicht nach einem der Ansprüche 1 bis 3, **dadurch gekennzeichnet, dass** die Öffnungen (4) eine Fläche zwischen 3 und 9 mm² aufweisen.

5. Flüssigkeitsverteilschicht nach einem der Ansprüche 1 bis 4, **dadurch gekennzeichnet, dass** die erste Gewirkelage (1) in einer Produktionsrichtung (P) zwischen 8 und 12 Maschenreihen pro Zentimeter (/cm) sowie in einer Querrichtung (Q) zwischen 7 und 10 Maschenstäbchen pro Zentimeter (/cm) aufweist.

6. Flüssigkeitsverteilschicht nach einem der Ansprüche 1 bis 5, **dadurch gekennzeichnet, dass** der Druckspannungswert CV₄₀ bei einer Verformung von 40% gemäß DIN EN ISO 3386-1 zwischen 4 und 8 Kilopascal (kPa) beträgt.

7. Flüssigkeitsverteilschicht nach einem der Ansprüche 1 bis 6, **dadurch gekennzeichnet, dass** die entlang einer Produktionsrichtung (P) gemäß EN ISO 13934-1 bestimmte Dehnung bei einer Zugkraft von 25 N weniger als 8 % beträgt.

8. Flüssigkeitsverteilschicht nach einem der Ansprüche 1 bis 7, **dadurch gekennzeichnet, dass** die erste Gewirkelage (1) und die zweite Gewirkelage (2) eine übereinstimmende Affinität zu Wasser aufweisen.

9. Flüssigkeitsverteilschicht nach einem der Ansprüche 1 bis 8, **dadurch gekennzeichnet, dass** das Flächengewicht zwischen 200 g/m² und 230 g/m² beträgt.

10. Flüssigkeitsverteilschicht nach einem der Ansprüche 1 bis 9, **dadurch gekennzeichnet, dass** die Abstandsfäden (3) von einem glatten Monofilamentgarn gebildet sind.

## Claims

1. A liquid-distributing layer for incontinence products with a first flat knit layer (1), with a second flat knit layer (2) having a pattern of openings (4) and with spacer threads (3) connecting the first knit layer (1) and the second knit layer (2) to one another, wherein the total thickness amounts to more than 3 mm and less than 6 mm, and wherein the openings (4) are arranged in a diamond pattern, **characterized in that** the density per unit of area amounts to less than 300 g/m², wherein the spacer threads (3) have a fineness between 80 dtex and 130 dtex and are arranged in a density between 65 and 110 per square centimeter (/cm²).

2. The liquid-distributing layer according to claim 1, **characterized in that** the density of the openings provided in the second knit layer is between 3 and 6.5 per square centimeter (/cm²).

3. The liquid-distributing layer according to claim 1 or 2, **characterized in that** the first and second knit layers (1, 2) and the spacer threads (3) are made of polyester threads.

4. The liquid-distributing layer according to any one of claims 1 to 3, **characterized in that** the openings (4) have an area between 3 and 9 mm².

5. The liquid-distributing layer according to any one of claims 1 to 4, **characterized in that** the first knit layer (1) has between 8 and 12 rows of stitches per centimeter (/cm) in one production direction (P) and has between 7 and 10 vertical rows of stitches per centimeter (/cm) in a transverse direction (Q).

6. The liquid-distributing layer according to any one of claims 1 to 5, **characterized in that** the compressive stress value CV₄₀ amounts to between 4 and 8 kilopascal (kPa) at a deformation of 40% according to DIN EN ISO 3386-1.

7. The liquid-distributing layer according to any one of claims 1 to 6, **characterized in that** the elongation determined along a production direction (P) according to EN ISO 13934-1 amounts to less than 8% at a tensile force of 25 N.

8. The liquid-distributing layer according to any one of claims 1 to 7, **characterized in that** the first knit layer (1) and the second knit layer (2) have a similar affinity for water.

9. The liquid-distributing layer according to any one of claims 1 to 8, **characterized in that** the density per unit of area is between 200 g/m² and 230 g/m².

10. The liquid-distributing layer according to any one of claims 1 to 9, **characterized in that** the spacer threads (3) are formed by a smooth monofilament yarn.

## Revendications

1. Couche de répartition de liquide pour produits d'incontinence, comportant une première couche de tissu superficielle (1), une seconde couche de tissu (2) présentant un motif d'orifices (4) et des fils d'espacement (3) qui relient ensemble la première couche de tissu (1) et la seconde couche de tissu (2), l'épaisseur totale étant de plus de 3 mm et de moins de 6 mm et les orifices (4) étant disposés suivant un motif de losanges, **caractérisée en ce que** le grammage est de moins de 300 g/m2, les fils d'espacement (3) présentant une finesse de 80 à 130 dtex et étant disposés en une densité de 65 à 110 par centimètre carré (/cm2).

2. Couche de répartition de liquide selon la revendication 1, **caractérisée en ce que** la densité des orifices pratiqués dans la seconde couche de tissu est de 3 à 6,5 par centimètre carré (/cm2).

3. Couche de répartition de liquide selon la revendication 1 ou 2, **caractérisée en ce que** la première et la seconde couche de tissu (1, 2), de même que les fils d'espacement (3), sont composées de files de polyester.

4. Couche de répartition de liquide selon une des revendications 1 à 3, **caractérisée en ce que** les orifices (4) présentent une surface de 3 à 9 mm2.

5. Couche de répartition de liquide selon une des revendications 1 à 4, **caractérisée en ce que** la première couche de tissu (1) présents dans un sens de production (P) 8 à 12 rangées de mailles par centimètre (/cm) et dans un sens transversal (Q) 7 à 10 colonnes de mailles par centimètre (/cm).

6. Couche de répartition de liquide selon une des revendications 1 à 5, **caractérisée en ce que** la valeur de la contrainte de compression CV40, pour une déformation de 40 % suivant la DIN EN ISO 3386-1, est de 4 à 8 kilopascal (kPa).

7. Couche de répartition de liquide selon une des revendications 1 à 6, **caractérisée en ce que** l'étirement défini le long d'un sens de production (P) suivant la norme EN ISO 13934-1 pour une force de traction de 25 N, est de moins de 8 % à.

8. Couche de répartition de liquide selon une des revendications 1 à 7, **caractérisée en ce que** la première couche de tissu (1) et la seconde couche de tissu (2) présentent une affinité concordante avec l'eau.

9. Couche de répartition de liquide selon une des revendications 1 à 8, **caractérisée en ce que** le grammage est de 200 g/m2 et 230 g/m2.

10. Couche de répartition de liquide selon une des revendications 1 à 9, **caractérisée en ce que** les fils d'espacement (3) sont composés d'un fil monofilament lisse.
